Europäisches Patentamt

**European Patent Office**

**Office européen des brevets**

(11) Publication number: **0 131 653**
**A1**

(12) # EUROPEAN PATENT APPLICATION

(21) Application number: **83201074.8**

(22) Date of filing: **19.07.83**

(51) Int. Cl.⁴: **A 61 B 17/22**
**G 10 K 11/30**

(43) Date of publication of application:
**23.01.85 Bulletin 85/4**

(84) Designated Contracting States:
**AT BE CH DE FR GB IT LI NL SE**

(71) Applicant: **N.V. Optische Industrie "De Oude Delft"**
**Van Miereveltlaan 9**
**NL-2612 XE Delft(NL)**

(72) Inventor: **Mulder, Hendrik**
**Hendrik Marsmanlaan 7**
**2624 TJ Delft(NL)**

(72) Inventor: **Duinker, Simon**
**Mollaan 1**
**2061 CR Bloemendaal(NL)**

(74) Representative: **Urbanus, Henricus Maria, Ir. et al,**
**c/o Vereenigde Octrooibureaux Nieuwe Parklaan 107**
**NL-2587 BP 's-Gravenhage(NL)**

(54) Apparatus for the non-contact disintegration of stony objects present in a body by means of sound shockwaves.

(57) Apparatus for the non-contact disintegration of concrements present in a body, in which sound shockwaves generated in a liquid are focussed on to a concrement to be disintegrated, and in which, for the focussing, there is provided a positive acoustic lens (11) or lens configuration placed in liquid.

FIG.1

EP 0 131 653 A1

Croydon Printing Company Ltd.

Title:

Apparatus for the non-contact disintegration of stony objects
present in a body by means of sound shockwaves .

----

This invention relates to an apparatus for the non-contact

disintegration of stony objects present in a body (hereinafter referred to as concrements), in which sound shockwaves generated in a liquid are focussed on to a concrement to be disintegrated.

A similar apparatus is known, for example, from German Offen- legungsschrift 2,351,247, which describes an apparatus comprising a semi-elliptical reflector. In the first focus of the ellipse, located within the reflector, sound shockwaves can be generated by spark discharge, which shockwaves are focussed by the reflector in the second focus of the ellipse. If this other focus coincides with a concrement to be treated the focussed sound shockwaves lead to disintegration of the concrement.

One disadvantage of this prior apparatus is that, in positioning the elliptical reflector, there is no degree of freedom whatsoever.

If, for example, as a result of the location of a concrement and/or the configuration of ancillary apparatus, it is not possible with a given reflector to cause the second focus to coincide with the concrement, it is necessary to choose another reflector, because, as it is, the distance between the two foci is fixed in an ellipsoidal reflector.

It is an object of the present invention to overcome this disadvantage.

For this purpose, according to the invention, an apparatus of the kind described is characterized in that, for the focussing, there is provided a positive acoustic lens or lens configuration placed in liquid.

The invention will be described in more detail hereinafter with reference to the accompanying drawings of a few embodiments.

Fig. 1 illustrates diagrammatically the basic idea of the present invention;

Fig. 2 shows diagrammatically a practical embodiment of the invention;

Fig. 3 shows a variant of Fig. 2;

Fig. 4 shows another variant of Fig. 2; and

Fig. 5 shows still another variant.

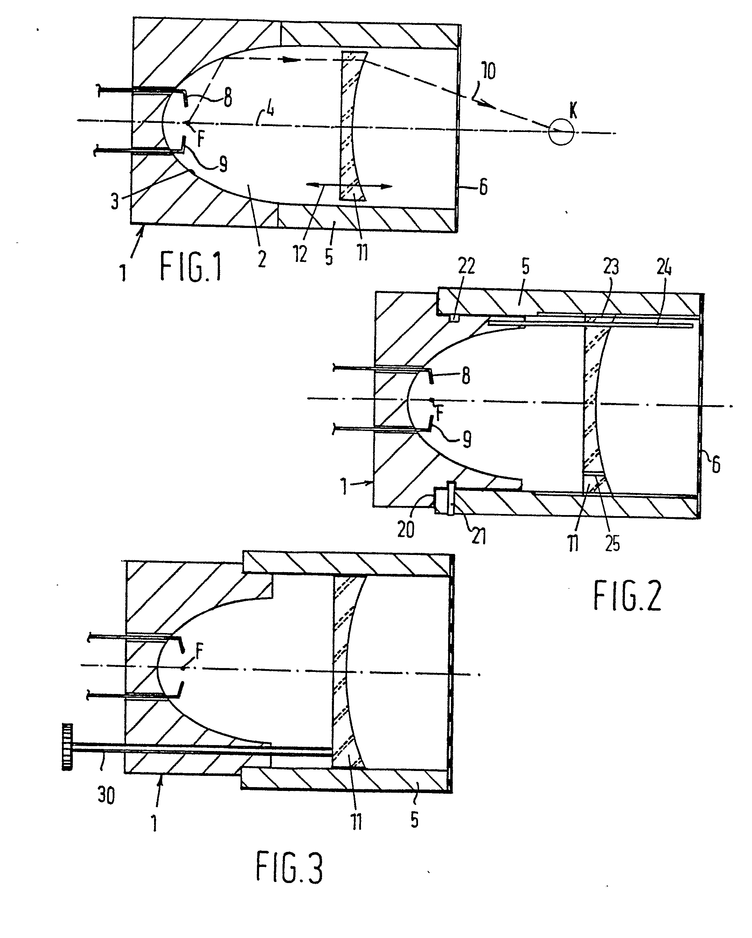

Fig. 1 shows a reflector block 1, which for example may be cylindrical, and in which a reflector cavity is formed, whose inner surface forms a reflector 3 for ultrasound with a parabolic cross-sectional configuration (parallel to the plane of the page). The reflector cavity may be rotationally symmetrical about the axis 4, or may be parabolic in any cross-section parallel to the plane of the page, so that a focal line is formed. The reflector will hereinafter be referred to as a parabolic reflector, which term should be construed as convering both of the above embodiments. The open end of reflector block 1 is contiguous with one end of a cylindrical extension piece 5, which if necessary is provided on the inside with a material which absorbs or diffuses sound waves, and is closed at its other end with a membrane 6. In operation, this membrane can be held in contact with a patient's skin, if desired using a jelly-like or fatty substance.

The reflector cavity and the extension piece 5 are filled with a liquid.

The reflector may be mounted in the bottom or sidewall of a bath filled with liquid, in which a patient can be placed. In that case it is not necessary to use a membrane, unless the reflector cavity and the extension piece should be filled with a different liquid from the bath. Also, in that case, depending on the configuration selected, the extension piece may be omitted in part or altogether. In operation, ultrasonic sound shockwaves are generated in the focus F of the parabolic reflector. For this purpose, in this example, two electrodes 8,9 are shown, between which a spark discharge can be effected. It is also possible, however, to generate sound shockwaves by other means, for example, with the help of an ultrasonic transducer.

The shockwaves generated in focus F, after reflection against the parabolic reflector, form a plane wave front as shown diagrammatically at 10.

This plane wave front should be focussed on to the concrement K to be disintegrated. For this purpose, according to the invention, a positive acoustic lens or lens configuration 11 is used, which is placed in the plane wave path, and in the embodiment shown, is disposed in the extension piece 5. The lens is slidable in the direction of axis 4, as indicated by an arrow 12, to permit accurate focussing.

The lens should be made of such a material that, at the transitions liquid-lens and lens-liquid, the reflection losses are minimal. In other words, the lens should preferably have the same acoustic impedance as the liquid. Suitable combinations of lens material and liquid are known from ultrasonic technology.

Fig. 2 diagrammatically shows a practical embodiment of the apparatus of Fig. 1. Extension piece 5 is coupled with the reflector block 1 for relative rotation. For this purpose the reflector block is provided on its outside with an annular shoulder 20, against which lies one end of the extension piece 5, which at least at that point is of right-cylindrical configuration. The extension piece is further provided with at least one cross-bore, through which extends a locking pin 21 into an annular groove 22 in the reflector block, to prevent the extension piece from sliding off the reflector block.

The lens is provided at its circumference with screwthread, which cooperates with inner screwthread formed in the extension piece, as shown at 23.

Furthermore, the lens is locked from rotation by a pin 24, extending through an opening adjacent to the rim of the lens, and secured in the reflector block, and if necessary a liquid passage 25 is provided in the lens.

By turning the extension piece relatively to the reflector block, and hence relatively to the lens, the lens is moved along the axis, and the focus of the lens can be accurately positioned.

Naturally, the lens may be mounted in a frame, which is then provided with outer screwthread cooperating with the inner screwthread of the extension piece.

If the reflector is secured in the wall of a bath, the extension piece may also be secured in the bath in a suitable manner.

As a variant of the embodiment of Fig. 2, the lens may be arranged to be slidable relatively to the extension piece, in which embodiment the extent of movement may be controlled by means of a threaded spindle extending outwards through a bore in the reflector block, which spindle cooperates with screwthread in the bore through the reflector block and is coupled to the lens.

Such a variant is shown in Fig. 3, in which the same reference numerals are used as in Fig. 2. The threaded spindle is designated by 30.

In another variant, not shown, the extension piece may be provided at the part surrounding the reflector block with inner screwthread, which cooperates with a corresponding screwthread on the reflector block. In that embodiment the lens may be fixedly mounted in the extension piece.

Instead of using a reflector and a point source of ultrasonic waves, it is possible to use a flat or curved ultrasound transducer with an associated acoustic lens or lens configuration.

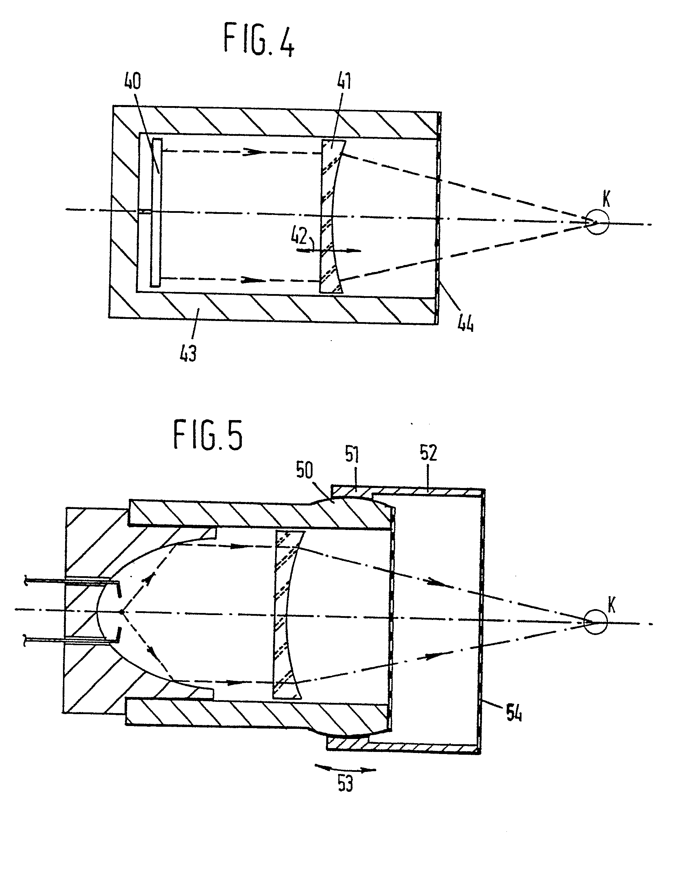

Such an embodiment is shown diagrammatically in Fig. 4. A transducer 40 having a relatively large diameter of, for example, 10 to 15 cm generates, after being energized, a shockwave, in this example a plane shockwave, which is focussed onto a concrement K by means of a suitable acoustic lens 41, which is slidable relatively to the transducer according to the arrow 42. The transducer and the lens are housed, for example, in a cylindrical housing 43 filled with liquid.

At the end away from the transducer, the housing may again be closed with a membrane 44 that can be held in contact with a patient. This, however, is not strictly necessary if use is made of a bath, as described before.

The slidability of the lens may be accomplished in the manner shown in Fig. 3, but also as shown in Fig. 2. In that case the housing should be made of two parts which are rotatable relatively to each other, analogously to the structure shown in Fig. 2.

It is noted that the embodiments described hereinbefore may be modified in various ways.

Thus, if the extension piece, or the housing (Fig. 4) is closed with a membrane, a second membrane may be mounted within the extension piece or housing at some distance from that membrane, while between the membranes there is provided a liquid whose acoustic wave resistance is intermediate that of the liquid present in the remainder of the extension piece and the reflector cavity of in the remainder of the housing, and that of the body to be treated, all this as described in European patent application No. 83 201 075.5, filed by the same applicants on July 19, 1983.

Furthermore, the extension piece or the housing may be connected with a coupling member permitting the apparatus to be tilted relatively to the patient, as also described in the above European patent application No. 83 201 075.5.

For the sake of completeness, Fig. 5 shows an example of such an apparatus provided with a coupling member.

In the embodiment shown in Fig. 5, the extension piece (or the housing) is provided on the outside, adjacent to the end away from the reflector (or the transducer), which end may or may not be closed with a membrane, with a convex annular thickening 50, which cooperates with a concave annular portion 51 of a cylindrical coupling member 52 to form a ball joint permitting movement according to the arrow 53.

0131653

The coupling member is filled with liquid and closed with a membrane 54 that can be placed in contact with a patient's skin. These and similar modifications are considered to fall within the scope of the present invention.

*******

\* C L A I M S \*

1. Apparatus for the non-contact disintegration of concrements present in a body, in which sound shockwaves generated in a liquid are focussed on to a concrement to be disintegrated, characterized in that, for the focussing, there is provided a positive acoustic lens or lens configuration placed in liquid.

2. Apparatus according to claim 1, characterized by a parabolic reflector formed in a reflector block, and means for generating sound shockwaves in the focus of said reflector, which after reflection against the reflector reach the lens or lens configuration as a plane wave front and are focussed by said lens or lens configuration.

3. Apparatus according to claim 2, characterized by the provision of means for adjusting the distance between the reflector and the lens or lens configuration.

4. Apparatus according to claim 3, characterized in that the lens or lens configuration is arranged in an extension piece contiguous with the open end of the reflector block, and that, for the purpose of adjusting the distance between the reflector and the lens or lens configuration, the extension piece is mounted on the reflector block for rotation with the lens being provided at its circumference with outer screwthread which cooperates with corresponding inner screwthread of the extension piece, the lens or lens configuration being locked from rotation by means of a locking pin extending through an opening in the lens or lens configuration.

5. Apparatus according to claim 2 or 3, characterized in that the reflector block is provided outside the reflector proper with a bore through which extends a threaded spindle connected with the lens or lens configuration for moving said lens or lens configuration within said extension piece.

6. Apparatus according to any one of claims 2-5, characterized in that, at the end remote from the reflector block, the extension piece is closed with a membrane.

7. Apparatus according to claim 6, characterized by the provision of a second membrane arranged within the extension piece in spaced relationship to the first membrane, and together with said first membrane and a portion of the extension piece forming a closed compartment containing a liquid having an acoustic impedance intermediate that of the liquid present in the remainder of the extension piece and that of the body.

8. Apparatus according to any one of claims 2-7, characterized in that the extension piece is connected at the end remote from the reflector block with a liquid-filled coupling member, which is closed with a membrane that can be placed into contact with the body.

9. Apparatus according to claim 8, characterized in that the extension piece and the coupling member are pivoted together.

10. Apparatus according to claim 1, characterized in that, for the generation of sound shockwaves, there is provided a transducer of large cross-section, which when energized generates sound shockwaves in the liquid, which are focussed by the lens or lens configuration.

11. Apparatus according to claim 10, characterized in that means are provided for adjusting the distance between the transducer and the lens or lens configuration.

12. Apparatus according to claim 10, characterized in that the transducer is disposed in the first part of a housing consisting of two parts disposed in alignment with each other, with the lens or lens configuration being disposed in the second part, said first part and said second part being rotatable relatively to each other with the second part being provided with inner screwthread cooperating with outer screwthread provided on the lens or lens configuration, there being provided means for locking the lens or lens configuration from rotation relative to the second part of the housing.

13. Apparatus according to claim 10, characterized in that the transducer and the lens or lens configuration are disposed in a housing which, in a rear wall located beyond the end of the transducer remote from the lens or lens configuration, is provided with a bore, through which extends a threaded spindle passing along the transducer and connected to the lens or lens configuration for adjustment of the distance between the transducer and the lens or lens configuration.

14. Apparatus according to any one of claims 10-13, characterized in that, at the end remote from the transducer, the housing is closed with a first membrane.

15. Apparatus according to claim 14, characterized by the provision of a second membrane within the housing, said second membrane being spaced some distance from the first membrane and, together with said first membrane and a portion of the housing, forming a closed compartment, which contains a liquid having an acoustic impedance intermediate that of the liquid contained in the remainder of the housing and that of the body.

16. Apparatus according to any one of claims 10-15, characterized in that, at the end away from the transducer, the housing is connected to a coupling member filled with liquid and closed with a membrane that can be placed into contact with the body.

17. Apparatus according to claim 16, characterized in that the housing and the coupling member are pivoted together.

*******

FIG.1

FIG.2

FIG.3

FIG.4

FIG.5

European Patent
Office

**EUROPEAN SEARCH REPORT**

**0131653**

Application number

EP 83 20 1074

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | WO - A - 82/04157 (LIERKE) | | A 61 B 17/22 G 10 K 11/30 |
| X | * Claim 1; figures; page 4, line 10 - page 5, line 15 * | 1 | |
| Y | | 2,3 | |
| A | | 6,7 | |
| | -- | | |
| Y | DE - B - 2 832 312 (FORSSMANN) | | |
| | * Column 6, lines 1,2; figure 1 * | 2,3 | |
| | -- | | |
| A | GB - A - 2 110 052 (KUMAR) | | |
| | * Figure 4, page 4, line 61 - page 5, line 8 * | 3,5 | |
| | -- | | |
| A | US - A - 3 934 460 (SHERWIN) | | |
| | -- | | |
| A | DE - A - 2 356 405 (NIPPON KOGAKU) | | |
| | * Figures 1,2 * | 4 | |
| | -- | | |
| A | DE - A - 2 712 051 (KATAGIRI) | | |
| | * Figures; claim 1 * | 4 | |
| | -- | | |
| | ./. | | |

TECHNICAL FIELDS
SEARCHED (Int. Cl. ³)

A 61 B
A 61 F
A 61 N
G 10 K
G 02 B

The present search report has been drawn up for all claims

| Place of search The Hague | Date of completion of the search 30-03-1984 | Examiner STEENBAKKER |
|---|---|---|

EPO Form 1503 03 82

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

0131653

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit The present European search report has been drawn up for the first ten claims and for those claims for which claims fees have been paid,

namely claims:

☐ No claims fees have been paid within the prescribed time limit. The present European search report has been drawn up for the first ten claims.

## ☒ LACK OF UNITY OF INVENTION

The Search Division considers that the present European patent application does not comply with the requirement of unity of invention and relates to several inventions or groups of inventions,
namely:

1. Claims 1-9: Disintegrating apparatus with parabolic reflector

2. Claims 1,10-17: Disintegrating apparatus with a transducer of large cross-section.

☐ All further search fees have been paid within the fixed time limit. The present European search report has been drawn up fo all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: 1 - 9

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| A | DE - A - 2 913 251 (WOLF) <br><br> * Claim 1, page 6, line 10 - page 7, line 11; figures * <br><br> ———— | 8,9 | |